(19) Europäisches Patentamt
European Patent Office
Office européen des brevets

(11) **EP 1 950 561 A1**

(12) **EUROPÄISCHE PATENTANMELDUNG**

(43) Veröffentlichungstag:
**30.07.2008  Patentblatt 2008/31**

(21) Anmeldenummer: 07001863.5

(22) Anmeldetag: **29.01.2007**

(51) Int Cl.:
*G01N 33/46* (2006.01)        *G01N 29/04* (2006.01)
*G01B 11/245* (2006.01)      *G01N 21/898* (2006.01)
*G01N 3/40* (2006.01)

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK RS**

(71) Anmelder: **LuxScan Technologies SARL**
**4384 Ehlerange (LU)**

(72) Erfinder:
• **Vogrig, Raphael**
  **4384 Ehlerange (LU)**
• **Hergott, Jean-yves**
  **4384 Ehlerange (LU)**

(74) Vertreter: **Degwert, Hartmut**
**Prinz & Partner GbR**
**Rundfunkplatz 2**
**80335 München (DE)**

(54) **Berührungslose Kontrolle der Festigkeit von Holzprodukten in der Produktionslinie**

(57)     Es wird ein ohne ionisierende Strahlung , in der Produktionslinie arbeitendes System zur Ermittlung der Fetsigkeitsklasse von Holzprodukten beschrieben, welches mit einem einfachen Wägsesystem zur Bestimmung des Rohgewichtes, einer Schwingungsanlage zur Bestimmung des E-Moduls und einem System von bildgebenden, das Produkt bildhafterfassenden Sensoren arbeitet, welche gleichzeitig anhand der Produktumrisse das Produktvolumen ermitteln und anhand der visuell erkennbaren, die Festigkeit beeinflussenden Eigenschaften wie Ästigkeit, Faserrichtung u.ä. nicht nur eine Gesamtfestigkeitsklasse für das gesamte Produkt, sondern auch eine Landkarte der lokalen Festigkeitsklasse ermitteln.

Besonders vorteilhaft ist die Verwendung von farbtüchtigen bildgebenden Sensoren mit der zusätzlichen Möglichkeit, neben der Festigkeitsklasse gleichzeitig auch die gesamte oder lokale ästhetische Qualität des Holzproduktes zu ermitteln.

Fig. 1

EP 1 950 561 A1

**Beschreibung**

Die Bestimmung der Festigkeit von Holzprodukten wie Kantholz, Schnittholz, Schwellen u.ä. welche für die Verwendung als tragende oder versteifende Elemente in Dachkonstruktionen , Bauten, Brücken u.ä. eingesetzt werden, ist gesetzlich vorgeschrieben und wird durch zahlreiche Normen wie z.B. die EN 338 geregelt. Das Deutsche Institut für Bautechnik (DIBt), Berlin, erteilt die erforderlichen Zulassungen für den Einsatz in verschiedenen Festigkeitsklassen aufgrund von der 100% Überprüfung und Sortierung der eingesetzten Holzteile mit zugelassenen Messverfahren .

Jüngste Unfälle von unter Schneelast zusammenbrechende Holzbinder-Tragekonstruktionen von Sporthallen habe die öffentliche Aufmerksamkeit auf die Verwendung ausreichend und zuverlässig auf ihre Festigkeit geprüfte Holzprodukte gelenkt und die Verantwortlichkeit der Produzenten solcher Holzprodukte betont.

Es sind zahlreiche Verfahren zur Überprüfung der Festigkeit von zumeist länglichen Holzprodukten wie roh zugesägtes oder gehobeltes Schnittholz in der Produktionslinie bekannt. Diese Verfahren verwenden zumeist mehrere kombinierte zerstörungsfreie Messverfahren, um die Endfestigkeit des Produktes aufgrund der gemessenen Daten anhand von mathematischen oder heuristischen oder statistischen Modellen vorherzusagen:

- die Biegesteifigkeit bzw. Biegeelastizität des Holzträgers wird durch relativ komplizierte Anordnungen von kraftausübenden Biegerollen gemessen und zusätzlich wird die Dichte und das Vorhandensein von lokalen Schwachstellen wie Astlöcher und Ästigkeit durch eine bildgebende Röntgenanlage gemessen ( siehe z.B. Euro-Grecomat der Fa. Gecon, Alfeld, www. Grecon.com).

- der Elastizitäts-Modul (E-Modul) wird über die Analyse der Eigenschwingungen anhand von mechanischer Schwingungsanregung gemessen, wobei die Schwingungen entweder kontaktbehaftet mit Schwingungsaufnehmer abgenommen werden oder berührungslos beispielsweise mit Laser-Vibrometer. Zusammen mit der Rohdichte kann hieraus auf die Festigkeit geschlossen werden.

Eine Firma verzichtet auf die Messung der Rohdichte (siehe z.B. das System Dynagrade der schwedischen Firma Dynalyse AB, www. Dynalyse.com).

- visuelle Kriterien der Oberfläche wie die folgenden, der DIN 4074-1 entnommenen Sortiermerkmale- und -kriterien:

- Ästigkeit

- Faserneigung

- Vorhandensein von Markröhren

- Breite der Jahresringe

- Vorhandensein von Rissen

- Vorhandensein von Baumkante

- Krümmung des länglichen Holzproduktes

- Verfärbung und Fäule

- Vorhandensein von Druckholz

- Vorhandensein von Insektenfraß

- sonstige Merkmale

wobei diese visuellen Merkmale heute noch vorwiegend manuell erfasst und beurteilt werden und damit einer grossen Streuung und Unsicherheit unterliegen aufgrund der wenig quantitativen und reproduzierbaren Arbeitsweise der menschlichen Prüfer.

Fast alle publizierten Verfahren der automatisierten Festigkeitssortierung in der Produktions-linie beschränken sich daher auf die physikalischen Eigenschaften wie Rohdichte, Schwingungs- und Biegungsverhalten und messen diese Eigenschaften mit berührungslosen oder zumindestens berührungsarmen Meßverfahren.

In "Non-destructive testing for assessing wood members in structures - a review " von R.J. Ross und R.F. Pellerin, General Technical Report FPGL-GTR-70, United States Department of Agriculture/Forest Services/Forest Products Laboratory werden die wichtigsten Verfahren zusammengefasst und mit einer ausführlichen Bibliographie begleitet.

Das US Patent US4852029 "Automated material classification apparatus and method" mit Priorität vom 17. Juni 1987 beschreibt eine Anordnung und ein Verfahren für eine Sortierung nach Festigkeiten ("stress grading" ) durch die Analyse mechanisch eingebrachter Schwingungen mit Hilfe eines motorisierten Schlaghammers ("impactor").

Die zahlreichen bekannten Verfahren und die hieraus entwickelten, kommerziell angebotenen Systeme für die Sortierung von Holzprodukten nach Festigkeitsklassen leiden allerdings trotz ihrer behördlichen Zulassung alle unter einer Reihe schwerwiegender Nachteilen:

1. die Systeme, welche für die Bestimmung der Rohdichte die RöntgenTechnik oder nukleare Strahler einsetzen sind allein durch ihre ionisierende und für den Menschen gefährliche Strahlung und den damit verbundenen gesetzlichen Auflagen wie Schutzabschirmung, geforderter Strahlenschutzbeauftrager im Betrieb usw. nicht nur in der Anschaffung sondern auch im laufenden Betrieb und bei der Entsorgung sehr teuer

2. bildgebende Röntgenverfahren, welche neben der Rohdichte auch zumindestens teilweise die Ästigkeit erfassen, leiden darunter, dass Äste nur in einer Projektions-richtung erkannt werden. Da aus Aufwandsgründen nur aus eine Richtung durchstrahlt wird, kann die Ausdehnung der Äste in Strahlungsrichtung nicht bestimmt werden.

Durch die Erfassung der Ästigkeit aus nur einer Richtung leidet die Zuordnung in Festigkeitsklassen erheblich, da gerade Äste als lokale Schwachstellen einen ganz erheblichen Einfluss auf die Festigkeit haben. Unsichere Ergebnisse werden aus Sicherheitsgründen immer in eine zu niedrige Festigkeitsklasse einsortiert und bedeuten daher einen erheblichen wirtschaftlichen Verlust für den Produzenten.

Rotierende Röntgen-Tomographen, wie sie in der EP 03012744 "Rotating Computer Tomograph", Anmelder Microtec srl beschrieben sind, können diesen Nachteil beheben, erhöhen allerdings nochmals signifikant den technischen Aufwand.

3. Da viele Holztragekonstruktionen in Innenräumen sichtbar sind, ist neben der Qualität der Festigkeit auch die visuelle ästhetische Qualität wichtig. Die Sortierung nach ästhetischen Kriterien wie Farbtönung, Bild der Maserung Vorhandensein von Kontaminationen usw. erfordern zusätzliche bildgebende , im optisch sichtbaren Bereich arbeitende Systeme welche heute, wenn überhaupt, getrennt von der Festigkeitssortierung mit menschlichen Prüfern oder separaten optischen Inspektionssystemen, oft mit Hilfe von Farbkameras, durch geführt werden (siehe z.B. Produkt MatchScan der Fa. LuxScan Technologies, www.luxscan.lu).

4. Ein weiterer Nachteil der bekannten Systemen ist es ebenfalls, dass sie in der Regel den gesamten Prüfling in eine einzige Festigkeitsklasse einordnen. Es gibt aber in vielen Prüflingen durchaus Abschnitte mit einer hohen Festigkeit, welche sich mit Abschnitten geringer Festigkeit abwechseln. Dies kann insbesondere dadurch auftreten, dass die o.g Merkmale aus der DIN 14074-1 entlang des Holzprüflings sehr unterschiedlich ausgeprägt ausfallen können.

Das mit einer bildgebenden Röntgenanalage versehene Festigkeitssortiersystem EuroGrecomat kann zwar lokal die von einer einzigen Seite aus sichtbare Ästigkeit erfassen, aber keine der übrigen visuellen Merkmale.

Beispielsweise dürfen die Ästigkeit , die Faserrichtung, das Vorhandensein von Markröhren und die Krümmung im Bereich der späteren Auflager einer Holztragekonstruktion nur sehr gering sein, in den übrigen Teilen aber durchaus höher. Eine automatisch erstellte "Landkarte" der lokalen Festigkeit würde damit eine bedeutend höhere Ausbeute von Schnittholz erlauben als eine lediglich summarische Klassifizierung in eine einzige Festigkeitsklasse.

Es wäre daher von hohem wirtschaftlichen Nutzen, die unterschiedlichen, der DIN Norm und äquivalenten Normen nach bestimmten Festigkeit eines Prüflings nicht nur in ihrer Gesamtheit sondern abschnittsweise entlang des länglichen Prüflings automatisch zu bestimmen. Besonders vorteilhaft wäre es, wenn ein solches System gleichzeitig mit der Festigkeitsanalyse auch die ästhetische Qualität messen könnte.

Es besteht daher wegen der genannten Nachteilen der derzeitigen Verfahren und Systemen ein erhebliches technisches und wirtschaftliches Interesse an einem kostengünstigen, berührungslosen, ohne gefährliche ionisierende Strahlungsquellen und automatisch in der Produktionslinie arbeitendem Verfahren und einer hierzu geeigneten Anordnung zur genauen Bestimmung der gesamten und der abschnittsweisen Festigkeitsklasse und der ästhetischen Qualität von Holzprodukten.

Dies wird erfindungsgemäß dadurch erreicht, dass die in der Festigkeit zu sortierenden Produkte durch mindestens drei in der Produktionslinie in beliebiger Reihenfolge und Gruppierung angeordneten ohne ionisierende Strahlung arbeitenden Meßsysteme ausgewertet werden, wobei über mindestens ein in der Produktionsstrasse eingebautes Meßsystem das Rohgewicht jedes Produktes ermittelt wird, durch die automatische Anregung von Schwingungen mit Hilfe mindestens eines Schwingungsgebers und die rechner-gestützte Analyse dieser akustisch und/oder optisch aufgenommenen Schwingungen die aus dem Schwingungsverhalten mit der Festigkeit korrelierenden Eigenschaften gemessen werden und durch eine Anordnung von das Holzprodukt aufnehmenden bildgebenden Sensoren gleichzeitig aus den im Bild sichtbaren Umrissen das Gesamtvolumen des Produktes sowie die optisch auf der Oberfläche des Prüflings erkennbaren, mit der Festigkeit korrelierende Eigenschaften gemessen werden und dass durch die Auswertung der von den mindestens drei Meßsystemen gelieferten Messdaten mit Hilfe eines physikalischen und/oder statistischen und/oder heuristischen Modells die Festigkeit des Produktes insgesamt und/oder die lokale Festigkeit in den verschiedenen Zonen des Produktes bestimmt werden.

Ein weiterer Erfindungsgedanke ist es, gleichzeitig mit der Bestimmung der Gesamtfestigkeit und/oder der lokalen Festigkeit des Produktes die Qualität des ästhetischen Eindrucks insgesamt und/oder die Landkarte der lokalen ästhetischen Qualität entlang des länglichen Produktes zu bestimmen indem die Bildsignale der bildgebenden Sensoren nach diesen Kriterien ausgewertet werden.

Der Erfindungsgedanke beschreibt damit ein sehr kostengünstiges Verfahren, welches keine aufwendige bildgebende Röntgentechnik erfordert und durch Einsatz kostengünstiger und berührungslos arbeitender Kameratechnik gleichzeitig die für die Qualität der Festigkeit und die Qualität des visuellen Eindrucks erforderlichen Informationen liefert. Durch die flächendeckende Erkennung aller Äste und der Faserstruktur werden wesentlich umfangreichere Informationen zur Ermittlung der Festigkeit geliefert als bei durchstrahlenen Röntgensystemen, welche die Ästigkeit lediglich von einer Seite erfassen. Die Sortierung in Festigkeitsklassen lässt sich damit viel genauer und mit geringerem Aufwand in der Produktionslinie durchführen sowie lokal als sog. Landkarte der lokalen Festigkeit durchführen.

Damit stellt dieses Verfahren und die zu seiner Durchführung geeigneten Anordnungen einen wesentlichen Fortschritt gegenüber dem beschriebenen Stand der Technik dar.

Der Erfindungsgedanke sei beispielhaft am Beispiel der Sortierung von Kantenholz in lokale Festigkeits- und ästhetische Klassen erläutert. Hierfür werden folgende Abbildungen verwendet:

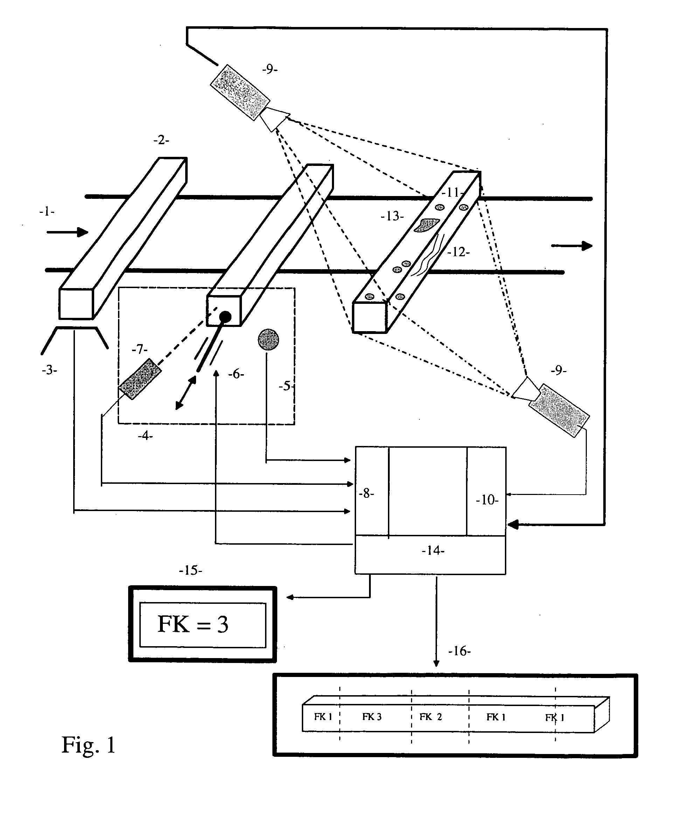

Fig. 1 zeigt eine beispielhafte Anordnung von im Quertransport -1-bewegten Kanthölzer -2- , welcher über die Bandwaage -3- gewogen werden, danach in eine Vibrationsanalyse-Station -4- durchlaufen, in welche über einen sensor-gesteuerten -5- Schlaghammer -6- Längsschwingungen angeregt werden und berührungslos über ein Laser-Vibrometer -7- detektiert und in einem Analyseeiheit -8- ausgewertet werden, danach durch eine Anordnung von zwei das Kantholz von allen Seiten bildgebend erfassenden Kameras -9- durchlaufen und mit einem Bildrechner -10- aus den Kamerabildern das Volumen des Kantholzes , die allseitige lokale Ästigkeit -11- und die lokale Faserstruktur -12- sowie das Vorhandensein von ästhetischen Fehlern -13- erfassen und dass in einer Auswertstation -14- sowohl eine für den gesamten Prüfling gültige Festigkeitsklasse -15- als auch eine Karte -16- der lokalen Festigkeitsklassen bestimmt, angezeigt und ausgegeben werden.

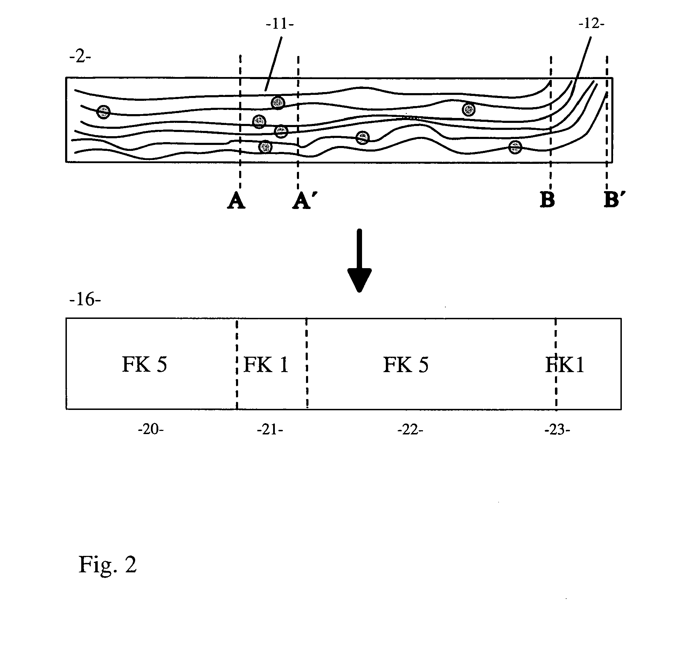

Fig.2 zeigt beispielhaft einen Holzprüfling mit vier ausgeprägten unterschiedlichen Festigkeitsklassen FK1 bis FK 5, welche durch eine lokal sehr unterschiedliche Ästigkeit -11- und Faserstruktur -12- bestimmt werden.

Das in Festigkeitsklassen und optional zusätzlich in ästhetische Klassen zu sortierende Kantholz wird nach Fig. 1 im Quertransport an drei Meßstationen vorbeigeführt. Diese sind aus Gründen der Anschaulichkeit nacheinander angeordnet. Der Erfindungsgedanke umfasst auch die Integration aller drei Verfahren an einem oder zwei Messorte, da die eingesetzten Verfahren sich gegenseitig wenig beeinflussen.

Der Holzprüfling wird demnach an folgenden Messstationen vorbeigeführt:

1. an einer Bandwaage - 3-, welche das Gesamtgewicht des Kantholzes im Transport bestimmt

2. an einer Anlage -4- zur Bestimmung des Elastizitätsmoduls ( abgek. E-Modul )mittels mechanisch eingebrachter Schwingungen durch einen motorisch bewegten Anschlag -6-, welcher durch einen Näherungssensor -5- ausgelöst wird. Die mechanischen Schwingungen werden berührungslos mit einem Laservibrometer -7- gemessen und zur Bestimmung des einem bestimmten Schwingungstyp zugeordneten E-Moduls an den Analyserechner -8-weitergeleitet.

3. einer optischen Station mit zwei Kameras -9-, welche das Kantholz auf allen vier Seitenflächen bildgebend erfassen, die Bildsignale an den Bildrechner - 10- weiterleiten wo mittels Verfahren der automatischen Bildauswertung und Mustererkennung folgende Eigenschaften des Holzprüflings berechnet werden:

a) das Volumen des Kantholzes anhand der optisch sichtbaren Kanten

b) die lokale Ästigkeit (Vorhandensein von Ästen) anhand der Muster, durch welche sich die Äste vom gesunden Holz unterscheiden

c) die Struktur der Holzfaser und Jahresringe

d) die übrigen die Festigkeit beeinflussenden visuellen Eigenschaften wie in dem og. Auszug aus der DIN 14074-1 genannten Merkmale

d) evtl. vorhandene ästhetische Kontaminationen wie Ölflecke oder farbliche Abweichungen. Im letzteren Fall werden für die Kameras -9- farbtaugliche Kameras eingesetzt.

Erfindungsgemäß wird aus dem Kamera-bestimmten Volumen und dem Gesamtgewicht die Rohdichte ermittelt, aus der Rohdichte , dem E-Modul und den norm-gemäßen visuellen Eigenschaften die Grundfestigkeit.

Weiterhin wird die lokale Festigkeitsklasse in den verschiedenen Längsabschnitten des Kantholzes dadurch bestimmt, dass beispielsweise die lokale Ästigkeit und die lokale Faserstruktur als Festigkeits-beeinflussende Eigenschaften in ein analytisches oder empirisches Modell.

$$\text{Festigkeitsklasse (Ort)} = f \left( \text{E-Modul, Grunddichte, Ästigkeit, Faserrichtung, Ort} \right) \quad /1/$$

eingegeben werden, welches fortlaufend auf dem Auswerterechner - 14- berechnet wird .

Die Beschränkung auf die beiden visuellen Merkmale "Ästigkeit" und "Faserrichtung" ist lediglich beispielhaft zu verstehen und wird aus Gründen der vereinfachten Erklärung des Erfindungsgedanken getroffen. Erfindungsgemäß werden alle mit bildgebenden Sensoren nach dem Stand der Technik erfassbaren visuellen, die Festigkeit beeinflussenden Merkmale gemessen . Hierzu können auch sog. multi-sensorielle bildgebende Sensoren eingesetzt werden , welche gleichzeitig mehrere visuelle Eigenschaften messen. ( siehe z.B. Robert Massen: The Multisensorial Camera for Industrial Apllications. Handbook of Computer Vision and Applications, Vol.1, pp.425-437, ed. Bernd Jaehne, Academic Press 1999).

Solche Modelle sind dem Fachmann der Festigkeitsanalyse bekannt, so z.B. aus :Diebold R. et al. : "Machine grading of structural sawn timber from various Softwood and Hardwood species". Proc. 12th Intern. Symposium on Nondestructive Testing of Wood, Sopron, 13-15.9.2000, pp. 139-146.

Die erfindungsgemässe Möglichkeit, nach Fig. 2 einen Prüfling nicht nur in eine Gesamt-Festigkeitsklasse zu sortieren, sondern eine "Landkarte" -16- der lokalen Festigkeit zu ermitteln, bedeutet eine wesentlich bessere Nutzung und damit eine höhere Wirtschaftlichkeit der nach Festigkeitsklassen sortierten Hölzer.

Im gegebenen Beispiel zeigt die "Landkarte" der Festigkeitsklasse vier verschiedene Abschnitte -20- bis -23- mit den jeweiligen individuellen Festigkeitsklassen FK1 und FK5. Hierbei soll FK5 die Klasse höchster und FK1 die Klasse mit der geringsten Festigkeit bezeichnen. Aufliegende Enden einer Holztragekonstruktion erfordern die höchste Klasse FK5 da hier Lagerkräfte durch den Prüfling aufgenommen werden müssen. Die lokale Stelle A-A' ist durch eine hohe Anzahl von Ästen geschwächt, die Stelle B-B' durch eine ungünstige Faserrichtung (in Richtung der Lagerkräfte). Beide werden in die niedrige Festigkeitsklasse FK1 eingeordnet. Damit ist dieser Prüfling insgesamt der Festigkeitsklasse FK1 zuzuordnen, d.h. er kann nicht als an beiden Enden gelagertes Trageelement verwendet werden.

Durch Auftrennen und Ausschneiden an die Stelle A-A' und B-B' können hingegen zwei hochfeste Tragebalken gewonnen werden , wobei nur wenig Holzverlust entsteht. Damit entstehen zwei hochfeste und tragfähige Balken, welche in Konstruktionen mit kürzerer Länge eingesetzt werden können. Die erfindungsgemässe Landkarte der lokalen Festigkeit erlaubt daher ein wesentlich höhere Holzausbeute und Wirtschaftlichkeit.

Ein weiterer Erfindungsgedanke ist es, mittels der Kameraanordnung -9- nicht nur die Geometrie ( das Volumen ) und die die Festigkeit beeinflussenden lokalen Merkmale "Ästigkeit" und "Faserrichtung" zu erfassen, sondern auch ästhetische Eigenschaften wie Farbtönung, Vorhandensein von Verschmutzungen und ähnliche, die Festigkeit nicht betreffende, aber den visuellen Eindruck beeinflussende Merkmale zu erfassen. Da zahlreiche Holztragekonstruktionen nicht überlackiert sind sondern im Gegenteil der ästhetische Holzeindruck ein gestalterisches Element darstellt, ist es von grosser wirtschaftlicher Bedeutung, auch die Holzästhetik einer Tragekonstruktion sicherzustellen.

Die Verwendung von Kameras, insbesondere von Farbkameras zur Sortierung nach ästhetischen Kriterien ist dem Fachmann der Bildverarbeitung bekannt und braucht daher nicht mehr detailliert zu werden (siehe z.B. Robert Massen et. al. "Automatic Inspection and Sorting of Laminates in the Production Line with MultiSensorial Vision Technology"; Pan-American Laminates Conference and Workshop, Montreal ,Canada, 4-6 octobre 2005, ed. TCM, Wien, www.tcm.at ).

Das erfindungsgemäße Verfahren der gleichzeitigen Bestimmung der lokalen Festigkeit UND ästhetischen Qualität bedeutet einen hohen wirtschaftlichen Vorteil, weil frühzeitig in der Produktionsstufe eine Schnittoptimierung nach diesen beiden Kriterien erfolgen und die Ausbeute signifikant erhöht werden kann.

Es ist ein weiterer Erfindungsgedanke, die Kameras -9- als "multi-sensorielle" Kameras auszulegen, um gleichzeitig die geometrischen und die Oberflächeneigenschaften zu messen (siehe SICK-IVP multiscan camera,www.sik-kivp.se).

Die beschriebene Anordnung ist lediglich beispielhaft zu verstehen. Die Anwendung von mehr als zwei Kameras, der Transport in Längs- statt in Querrichtung, die Gruppierung der drei eingesetzten Meßverfahren "Wiegen" , "Schwingungsanalyse" und "Bildauswertung" kann ebenso in einer Einheit oder in zwei Einheiten erfolgen, ohne den Erfindungsgedanken zu verlassen.

**Patentansprüche**

1. Verfahren zur kostengünstigen automatischen Sortierung in der Produktionslinie von Produkten, insbesondere aus Holz , in Klassen definierter Festigkeit ohne ionisierende Strahlung, **dadurch gekennzeichnet daß** die in der Festigkeit zu sortierenden Produkte durch in der Produktionslinie in beliebiger Reihenfolge und Gruppierung angeordnete mindestens drei Meßsysteme ausgewertet werden, wobei über mindestens ein in der Produktionsstrasse eingebautes Meßsystem das Rohgewicht jedes Produktes ermittelt wird, durch die automatische Anregung von Schwingungen mit Hilfe mindestens eines Schwingungsgebers und die rechner-gestützte Analyse dieser akustisch und/oder optisch aufgenommenen Schwingungen die aus dem Schwingungsverhalten mit der Festigkeit korrelierenden Eigenschaften gemessen werden und durch eine Anordnung von das Holzprodukt aufnehmenden bildgebenden Sensoren gleichzeitig aus den im Bild sichtbaren Umrissen das Gesamtvolumen des Produktes sowie die optisch auf der Oberfläche des Prüflings erkennbaren, mit der Festigkeit korrelierenden Eigenschaften gemessen werden und dass durch die Auswertung der von den mindestens drei Meßsystemen gelieferten Messdaten mit Hilfe eines physikalischen und/oder statistischen und/oder heuristischen Modells die Festigkeit des Produktes insgesamt und/oder die lokale Festigkeit in den verschiedenen Zonen des Produktes bestimmt werden.

2. Verfahren nach Anspruch 1, **dadurch gekennzeichnet daß** gleichzeitig mit der Bestimmung der Gesamtfestigkeit und/oder der lokalen Festigkeiten des Produktes die Qualität des ästhetischen Eindrucks insgesamt und/oder die lokalen ästhetischen Qualitäten entlang des länglichen Produktes bestimmt werden.

3. Verfahren nach Anspruch 1 bis 2, **dadurch gekennzeichnet, daß** das Meßsystem zur Bestimmung der Rohdichte eine Waage enthält.

4. Verfahren nach Anspruch 1 bis 3, **dadurch gekennzeichnet daß** durch Division des Rohgewichtes mit dem vom System bildgebender Sensoren ermittelten Raumvolumen des Produktes die Rohdichte des Produktes ermittelt wird.

5. Verfahren nach Anspruch 1 bis 4, **dadurch gekennzeichnet, daß** die bildgebenden Sensoren farbtüchtige Sensoren sind.

6. Verfahren nach Anspruch 1 bis 5, **dadurch gekennzeichnet, daß** die Produkte nach den Kriterien der Gesamtfestigkeit und gesamten ästhetischen Qualität und/oder der lokalen Festigkeit und lokalen ästhetischen Qualität sortiert werden.

7. Verfahren nach Anspruch 3, **dadurch gekennzeichnet, daß** die Produkte nach den Kriterien der lokalen Festigkeit und/oder der lokalen ästhetischen Qualität in Abschnitte mit vorgegebener Festigkeit und/oder ästhetischen Qualität zugeschnitten werden.

8. Verfahren nach Anspruch 1, **dadurch gekennzeichnet, daß** die mit der Festigkeit korrelierenden optisch sichtbaren Eigenschaften durch multi-sensorielle bildgebende Sensoren ermittelt werden, welche gleichzeitig mehrere mit der Festigkeit korrelierende Eigenschaften bildhaft ermitteln.

9. Anordnung zur Durchführung des Verfahrens nach Anspruch 1, **dadurch gekennzeichnet daß** die Produkte, deren Festigkeitsklasse insgesamt und/oder abschnittsweise bestimmt werden soll, über eine Transportwaage im Durchlauf gewogen werden, dass über eine Anordnung aus mechanischem Schwingungsanreger und akustisch und/oder optischem Schwingungsabnehmer das Schwingungsverhalten des Produktes gemessen wird, dass über ein System von mindestens zwei geometrisch kalibrierten Kameras die Umrisse des Produktes optisch erfasst und hieraus das Rohvolumen bestimmt wird, dass durch eine oder mehrere Rechnereinheiten aus der Division von Rohvolumen und Rohgewicht die Rohdichte des Produktes ermittelt wird und dass aus den Werten des Schwingungsverhaltens und der Rohdichte der die Festigkeit wesentlich bestimmenden Elastizitätsmodul berechnet wird und dass diese Daten des Prüflings durch Vergleich mit rechner-basierten Referenzdaten und/oder Modellen die Festigkeitsklasse bestimmt werden.

10. Anordnung nach Anspruch 9, **dadurch gekennzeichnet daß** aus den Bildern der bildgebenden Sensoren mit einem Bildrechner und Mustererkennungsverfahren die lokalen, die Festigkeit beeinflussenden visuell erkennbaren Eigenschaften wie Ästigkeit, Faserrichtung, Kantenausbrüche u.ä. ermittelt werden und diese Daten zur Bestimmung der Festigkeitsklasse insgesamt und/oder der zonenweisen Festigkeitsklasse des Produktes mit Hilfe des rechnerbasierten Modells bestimmt werden.

11. Anordnung nach Anspruch 9 bis 10, **dadurch gekennzeichnet daß** aus den von den Bildern der vorwiegend farbtüchtigen bildgebenden Sensoren mit einem Bildrechner und Mustererkennungsverfahren die ästhetische Qualität des Produktes insgesamt oder zonenweise bestimmt wird.

12. Anordnung nach Anspruch 9 bis 11, **dadurch gekennzeichnet, daß** aus den rechner-basierten Daten bezüglich der gesamten und/oder lokalen Festigkeit Sortiersignale zur Sortierung des gesamten Produktes erzeugt werden und/oder Signale und/oder Markierungen auf dem Holzprodukt erzeugt werden, welche eine automatische Auftrennung mit einer Auftrennmechanik des Produktes in Zonen gleicher Festigkeit und/oder gleicher ästhetischer Qualität steuern.

Fig. 1

-11-

-12-

-2-

A    A'    B    B'

-16-

| FK 5 | FK 1 | FK 5 | FK1 |
|------|------|------|-----|

-20-    -21-    -22-    -23-

Fig. 2

**Europäisches Patentamt**

**EUROPÄISCHER RECHERCHENBERICHT**

Nummer der Anmeldung

EP 07 00 1863

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 42 23 787 A1 (DIMTER MASCHF GMBH [DE]) 20. Januar 1994 (1994-01-20) | 1,3,4, 8-10,12 | INV. G01N33/46 |
| Y | * das ganze Dokument * | 2,5-7,11 | G01N29/04 G01B11/245 |
| D,Y | LUXSCAN TECHNOLOGIES: "Match-Scan M100/M200" LUXSCAN-PRODUKTKATALOG, [Online] 2006, XP002445713 [gefunden am 2007-08-06] * das ganze Dokument * | 2,5-7,11 | G01N21/898 G01N3/40 |
| L | TECHNOPORT NEWS, Nr. 10, Juli 2006 (2006-07), XP002445805 * Seite 4 * | | |
| A | WO 2004/106918 A (WEYERHAEUSER CO [US]) 9. Dezember 2004 (2004-12-09) * Seite 26, Zeilen 9-32; Anspruch 1; Abbildung 1 * | | |
| D,A | US 4 852 029 A (POPE THOMAS A [US] ET AL) 25. Juli 1989 (1989-07-25) | | |
| A | KUHWEIDE, WAGNER, WIEGAND: "Folge 3: Vollholzprodukte" HOLZBAU HANDBUCH REIHE 4, Bd. 2, Juni 2000 (2000-06), XP002445714 Düsseldorf ISSN: 0466-2114 * Absatz [05.5] * | | |
| D,A | ROBERT MASSEN: "Chapter 16: The multisensorial camera for industrial vision applications" HANDBOOK OF COMPUTER VISION AND APPLICATIONS; VOL. 1 SENSORS & IMAGING, Bd. 1, 1999, Seiten 425-437, XP008082116 ISBN: 0-12-379771-3 * Absatz [16.2] * | | |

RECHERCHIERTE SACHGEBIETE (IPC)

G01N
G01B

-/--

Der vorliegende Recherchenbericht wurde für alle Patentansprüche erstellt

| Recherchenort | Abschlußdatum der Recherche | Prüfer |
|---|---|---|
| Den Haag | 7. August 2007 | Kraus, Leonie |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

..........................................................................................

& : Mitglied der gleichen Patentfamilie, übereinstimmendes Dokument

EPO FORM 1503 03.82 (P04C03)

**Europäisches**
**Patentamt**

**Nummer der Anmeldung**

EP 07 00 1863

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| D,A | R. DIEBOLD, A. SCHLEIFER, P. GLOS: "Machine Grading of Structural Sawn Timber from various Softwood and Hardwood Species" CONFERENCE PROCEEDINGS OF THE 12TH INTERNATIONAL SYMPOSIUM ON NONDESTRUCTIVE TESTING OF WOOD UNIVERSITY OF WESTERN HUNGARY, SOPRON, 13. September 2000 (2000-09-13), XP008082118 * Seite 141, Absatz 1 * ----- | | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| Den Haag | 7. August 2007 | Kraus, Leonie |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 07 00 1863

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-08-2007

| Im Recherchenbericht angeführtes Patentdokument | Datum der Veröffentlichung | Mitglied(er) der Patentfamilie | Datum der Veröffentlichung |
|---|---|---|---|
| DE 4223787 A1 | 20-01-1994 | AT 187105 T | 15-12-1999 |
| | | EP 0595438 A2 | 04-05-1994 |
| WO 2004106918 A | 09-12-2004 | AU 2004243873 A1 | 09-12-2004 |
| | | CA 2530919 A1 | 09-12-2004 |
| | | EP 1634070 A1 | 15-03-2006 |
| | | NZ 544435 A | 31-05-2007 |
| US 4852029 A | 25-07-1989 | CA 1326551 C | 25-01-1994 |
| | | WO 8810415 A1 | 29-12-1988 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4852029 A

- EP 03012744 A

**In der Beschreibung aufgeführte Nicht-Patentliteratur**

- The Multisensorial Camera for Industrial Apllications. **ROBERT MASSEN.** Handbook of Computer Vision and Applications. Academic Press, 1999, vol. 1, 425-437

- **DIEBOLD R et al.** Machine grading of structural sawn timber from various Softwood and Hardwood species. *Proc. 12th Intern. Symposium on Nondestructive Testing of Wood, Sopron,* 13. September 2000, 139-146